# EUROPEAN PATENT SPECIFICATION

(11) **EP 1 728 476 B1**
(45) Date of publication and mention of the grant of the patent: **18.04.2012**
(21) Application number: 06114585.0
(22) Date of filing: 26.05.2006
(51) Int. Cl.: A61B 17/11

(54) **Instrumentation for carrying out a gastrointestinal bypass**
Instrumentarium zur Durchführung eines Magenbypasses
Appareillage destiné a la réalisation d'une dérivation gastrique

(30) Priority: 27.05.2005 IT MI20051004
(43) Date of publication of application: 06.12.2006
(73) Proprietor: ETHICON ENDO-SURGERY, INC., Cincinnati, OH 45242-2839 (US)
(72) Inventor: Tacchino, Roberto, I-00144, Roma (IT); Bilotti, Federico, I-04011, Aprilia (Latina) (IT); D'Arcangelo, Michele, I-00142, Roma (IT); Pastorelli, Alessandro, I-00136, Roma (IT)
(74) Representative: Leihkauf, Steffen Falk

(56) References cited:
- WO-A-01/66020
- FR-A- 2 689 749
- US-A- 5 395 030
- US-A1- 2004 225 191
- US-A1- 2005 049 614
- US-B1- 6 543 456

## Description

In general terms, the present invention regards the instrumentation for carrying out a gastrointestinal bypass.

Such instrumentation is particularly adapted for being used intraluminally.

Techniques are known for carrying out gastrointestinal bypasses, included the gastric bypass known as "Roux-en-Y", following which the gastrointestinal course winds between a stomach pouch, to which a portion of the intestine is directly connected. A large part of the stomach and intestine is therefore bypassed with the creation of a gastroenteroanastomosis.

Such technique, however, has several drawbacks both in terms of high time required in execution and in terms of post-operative risks and complications.

As is known, in general both the laparoscopic and intraluminal approach considerably limit the drawbacks of the conventional surgical methodology. In particular, they permit limiting the invasiveness of the procedure, reducing the risks for the patient and shortening the post-operative course.

The currently available instrumentation does not however permit limiting the drawbacks of the prior art, and in particular of the gastrointestinal bypass techniques such as that described above. For example, the use of circular staplers, above all in the realisation of the gastroenteroanastomosis, requires large sections of the intestine, which comprise its integrity and continuity even before concluding the operation. It follows that the operations still results invasive and does not permit verifying the effectiveness and seal of the anastomosis, in particular of the enteroenteroanastomosis.

Other drawbacks of the known techniques and instrumentation are for example tied to the preliminary section of the intestine, which requires considerable experience to evaluate the correct length at which to carry out the cutting and to recognise the two sectioned flaps. A further drawback is instead due to the large area of action which substantially covers the entire patient abdomen.

WO 01/66020 A discloses a known apparatus and method for performing a bypass procedure in a digestive system using circular and linear stapler without specific guide means, the two part form of claim 1 is based on this document. US 6,543,456 discloses a method for minimally invasive surgery in the digestive system without specific guide means. US 5,395,030 A discloses a surgical device for stapling and fastening body tissues without guide wires or cables. US 2004/225 191 A discloses an endoscopic treatment system and anastomatic method using such a system.

The problem underlying the present invention is that of proposing instrumentation for carrying out gastrointestinal bypasses which are capable of overcoming the drawbacks mentioned with reference to the prior art, thus permitting satisfying the field's growing need to limit the risks and invasiveness.

Such problem is resolved by means of instrumentation for carrying out a gastrointestinal bypass in accordance with claim 1.

Further characteristics and advantages of the instrumentation for carrying out gastrointestinal bypasses according to the invention will be clear from the description reported below of preferred embodiments, given as indicative and not limiting, with reference to the attached figures, wherein:
Figure 1 illustrates a first step of the method, which does not form part of the invention, for carrying out a gastrointestinal bypass with the instrumentation of the present invention;
Figure 2 illustrates a second step of the method, which does not form part of the invention, for carrying out a gastrointestinal bypass with the instrumentation of the present invention;
Figures 3-6 illustrate several steps of the method, which does not form part of the invention, carried out with the instrumentation of the present invention and in particular the figure 5a illustrates an enlarged detail of figure 5 according to one exemplary anastomotic ring and from a different angle.

Figure 7 illustrates a possible embodiment variation of Figure 4.

In the following description and claims, any disclosure of surgical method features or reference to surgical methods do not form part of the claimed invention.

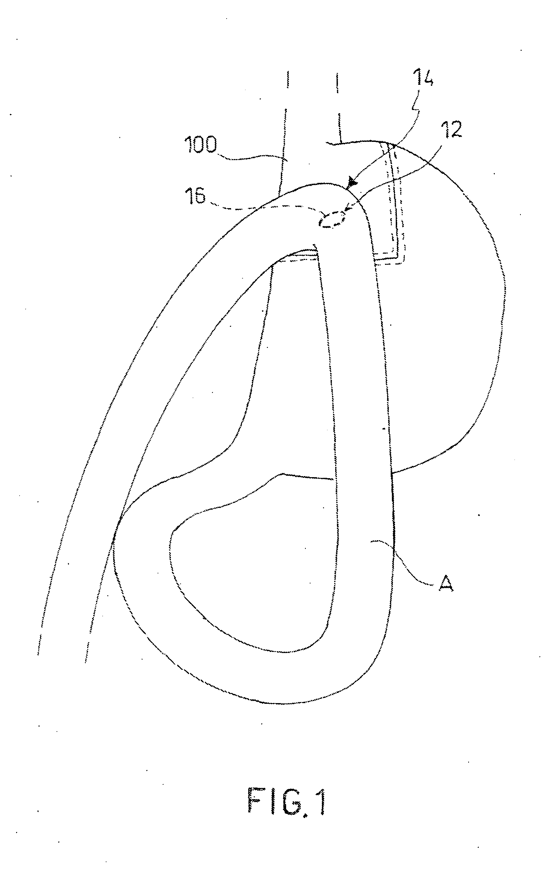

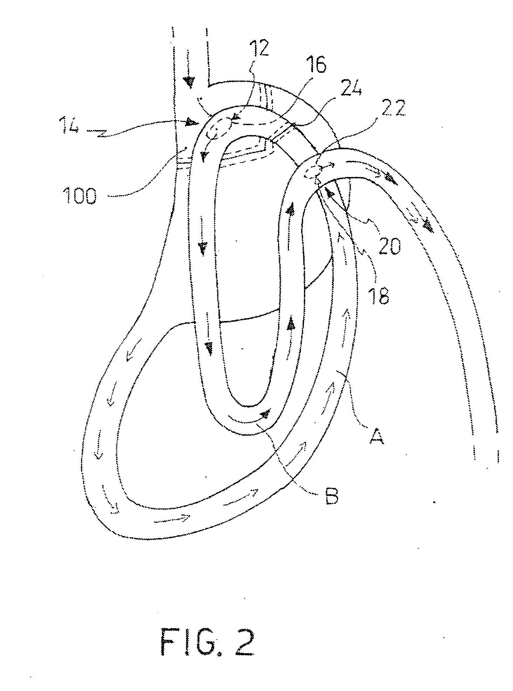

In accordance with one embodiment, the present invention regards to the instrumentation required for performing a method for carrying out gastrointestinal bypasses comprising the steps illustrated in figures 1 and 2.

This method or method steps do not form part of the invention.

Such exemplary method comprises steps of bringing close together and joining the tissues to form anastomoses adapted to maintain or restore the integrity and continuity of the intestinal duct after each anastomosis formation (whether gastroenteroanastomosis or enteroenteroanastomosis). Moreover, the gastroenteroanastomosis and the enteroenteroanastomosis are realised at close distances, balancing the contrasting needs to limit the operating zone, for example to only one upper zone of the abdomen, while keeping a wide operating and visual area.

In accordance with a possible exemplary method, a first step of the method foresees realising a gastric pouch 100 to which the first portion of the intestine will be connected. Subsequently, an area of the intestine may be selected to be united to the gastric pouch. The choice may be operated by means of measuring the available length and possibly verifying that tensions or distortions are not generated.

A first tissue portion 12 of the intestine, corresponding with the chosen area, is therefore brought close to a second tissue portion 14 of the stomach at the gastric pouch 100. A first loop A of the intestine is thus realised which extends between the stomach and the first tissue portion 12 of the intestine.

The two tissue portions may be slightly incised, forming an enterotomy and a gastrotomy to allow the insertion of respective jaws of a linear stapler. One jaw of the linear stapler may be inserted in the gastrotomy made at the second tissue portion of the stomach. The other jaw of the linear stapler may be inserted in the enterotomy made at the first tissue portion of the intestine. One flap of the two incisions may then be sutured by the linear stapler by means of a sequence of points, joining the two tissue portions and partially defining the gastroenteroanastomosis.

In accordance with a preferred exemplary method, the gastrotomy and enterotomy may be realised before bringing the two tissue portions close together. In this case, the linear stapler may be used as an instrument for bringing the two tissue portions close together, for example inserting one of the two jaws of the linear stapler into the enterotomy and using the stapler for bringing the first tissue portion close to the second tissue portion. The other jaw may then be inserted into the gastrotomy so to join the two tissues.

To complete the anastomosis (gastroenteroanastomosis 16) between the two tissue portions, the flaps of the gastrotomy and enterotomy may be reclosed, for example by means of suture. This allows restoring the continuity of the intestinal duct after the realisation of the gastroenteroanastomosis. Indeed, after having completed the gastroenteroanastomosis, the intestinal tract maintains its integrity and continuity, since the incisions carried out were restapled to form the gastroenteroanastomosis.

In accordance with a preferred exemplary method, the step wherein the gastroenteroanastomosis 16 is completed may substantially be realised at the end of the procedure, before sectioning the intestine and before carrying out the seal test, as will be described below.

Subsequently, an additional area of the intestine may be chosen, distal with respect to the gastroenteroanastomosis with reference to the natural flow along the intestinal duct, i.e. to the flow before carrying out the gastrointestinal bypass. In other words, by distal it is intended an area downstream of the gastroenteroanastomosis 16 with reference to the natural flow along the intestinal duct. The definition of proximal or distal will also be used below with reference to the natural flow inside the intestinal duct.

A corresponding additional first tissue portion 18 of the additional intestine area, distal with respect to the gastroenteroanastomosis 16 with reference to the natural flow along the intestinal duct, may then be brought close to an additional second tissue portion 20 of the intestine, proximal with respect to the natural flow along the intestinal duct, realising a second loop B of the intestine which is distal with respect to the gastroenteroanastomosis. In other words, as defined above, the additional first tissue portion 18 is arranged downstream of the gastroenteroanastomosis 16 with respect to the natural flow along the intestinal duct, moreover the additional second tissue portion 20 is arranged upstream of the gastroenteroanastomosis 16 with respect to the natural flow along the intestinal duct.

The additional second tissue portion 20 of the intestine, proximally arranged with respect to the gastroenteroanastomosis 16, may be brought close to the gastroenteroanastomosis.

At each of the two additional tissue portions 18, 20, an incision (enterotomy) may be made, adapted to receive a respective jaw of a linear stapler. A first jaw of the linear stapler may be inserted in the enterotomy made at the first tissue portion of the intestine. A second jaw of the linear stapler may be inserted in the enterotomy made at the additional second tissue portion of the intestine. A sequence of points may then be applied, which partially unites the flaps of the enterotomy and partially defines the enteroenteroanastomosis.

In accordance with a preferred example method described above, the enterotomies may be realised before bringing the two tissue portions close together. In this case, the linear stapler may be used as an instrument for bringing the two tissue portions close together, for example inserting one of the two jaws of the linear stapler into the enterotomy made at the additional first distal tissue portion, and using the stapler for bringing the first distal tissue portion close to the additional second proximal tissue portion. The other jaw may then be inserted in the enterotomy of the additional second proximal tissue portion so to join the two tissues.

The enteroenteroanastomosis 22 between the two tissue portions may subsequently be completed by reclosing the flaps of the enterotomies so to restore the continuity of the intestinal duct after the realisation of the enteroenteroanastomosis. The remaining flaps of the enterotomies may be joined for example by means of suture.

In accordance with a preferred exemplary method, the step wherein the enteroenteroanastomosis 22 is completed may be substantially realised at the end of the procedure, at the same time as the completion of the gastroenteroanastomosis before sectioning the intestine and before carrying out the seal test, as will be described below.

After having completed the enteroenteroanastomosis, the intestinal tract maintains its integrity and continuity, since the incisions carried out were restapled to form the enteroenteroanastomosis.

As illustrated in figure 2, the gastroenteroanastomosis and the enteroenteroanastomosis are rather close to each other, and permit operating in the upper part of the abdomen.

After having made the second loop, it is possible to carry out, preferably at the same time, a seal test of the two anastomoses, for example by means of methylene blue. The step of sectioning the intestine between the gastroenteroanastomosis and the enteroenteroanastomosis may be realised at the end of the procedure, when the correct functioning of the two anastomoses was verified. In figure 2, it is indicated with a section line 24.

Advantageously, the aforesaid method may be realised laparoscopically, comprising an initial step of inserting trocars, preferably four trocars arranged respectively in the following zones: epigastrium, left flank, and two trocars at the mesogastrium zone.

In accordance with the present invention, the instrumentation for carrying out a gastrointestinal bypass comprises:
- means for bringing a first tissue portion 12 of the intestine close to a second tissue portion 14 of the stomach, realising a first loop A of the intestine between the stomach and the first tissue portion of the intestine,
- means for creating a gastroenteroanastomosis 16 between the two portions of close tissue, maintaining or restoring the continuity of the intestinal duct after the realisation of the gastroenteroanastomosis,
- means for bringing close together an additional first tissue portion 18 of the intestine which is distally arranged or downstream with respect to the gastroenteroanastomosis 16 with reference to the natural flow along the intestinal duct, and an additional second tissue portion 20 of the intestine arranged proximally, or upstream, with respect to the natural flow along the intestinal duct, realising a second loop B of the intestine which is distal with respect to the gastroenteroanastomosis 16, wherein said additional second tissue portion 20 of the intestine arranged proximally with respect to the gastroenteroanastomosis 16 is brought close to the gastroenteroanastomosis 16,
- means for creating an enteroenteroanastomosis 22 between the two close tissue portions of the intestine, maintaining or restoring the continuity of the intestinal duct after the realisation of the enteroenteroanastomosis,
- means for sectioning the intestine between the gastroenteroanastomosis 16 and the enteroenteroanastomosis 22.

Preferably, the aforesaid instrumentation moreover comprises means for carrying out a seal test both of the gastroenteroanastomosis 16 and the enteroenteroanastomosis 22, before the intestine is sectioned between the gastroenteroanastomosis 16 and the enteroenteroanastomosis 22. Still more preferably, the means for carrying out the seal test are adapted to simultaneously test both the gastroenteroanastomosis 16 and the enteroenteroanastomosis 22. Such means may comprise, for example, means for inserting and visualising methylene blue through the intestinal duct.

In accordance with a possible exemplary instrumentation, which does not form part of the invention, the means for creating the gastroenteroanastomosis 16 may comprise a linear stapler adapted to partially join the flaps of a gastrotomy and enterotomy respectively made at the first portion of the intestine and the second portion of the stomach. The means for creating the gastroenteroanastomosis 16 may comprise moreover means for completing the gastroenteroanastomosis by reclosing the flaps still open after the use of the linear stapler, said means being adapted to restore the continuity of the intestinal duct after the realisation of the gastroenteroanastomosis. The linear stapling may also carry out the function of means for bringing close together the two tissue portions to be joined, inserting a jaw in the first tissue portion and using the stapler as a means for transporting the first tissue portion in correspondence with the second tissue portion.

In accordance with a possible exemplary instrumentation, which does not form part of the invention, the means for creating the enteroenteroanastomosis comprise a linear stapler adapted to partially join the flaps of enterotomies respectively made at the additional first and second portions of the intestine. Moreover, the means for creating the enteroenteroanastomosis may comprise means for completing the enteroenteroanastomosis by reclosing the flaps still open after the use of the linear stapler, said means being adapted to restore the continuity of the intestinal duct after the realisation of the enteroenteroanastomosis. Preferably, the linear stapling may also carry out the function of means for bringing close together the two tissue portions to be joined, inserting a jaw in the first tissue portion and using the stapler as a means for transporting the first tissue portion in correspondence with the second tissue portion.

The instrumentation according to the present invention advantageously comprises means for preliminarily realising a gastric pouch, wherein the second tissue portion 14 of the stomach is arranged at the gastric pouch.

Preferably, the means used are adapted to realise the gastrointestinal bypass laparoscopically.

The exemplary method and the instrumentation described above may be applied both to the step of carrying out the gastroenteroanastomosis and the step of carrying out the enteroenteroanastomosis, or to one of these.

Such method and instrumentation allow reducing the risks of mortality with gastrointestinal bypasses and considerably limit the operation times. Maintaining the continuity of the intestine until the completion of the two anastomoses, it is possible to verify both simultaneously. Moreover, due to the close arrangement of the two anastomoses, the operation area is limited to the upper zone of the abdomen.

Additionally, the advantageous prevision of forming two intestine loops without previously interrupting the continuity allows choosing the correct length, so to avoid tensions. Moreover, the realisation of the gastroenteroanastomosis without preliminary sectioning of the area permits reducing the risk of incorrectly joining the segments or inducing undesired torsions.

Beyond the above mentioned advantages, the use of the exemplary linear stapler permits limiting the size and extension of the bleeding, the losses and risk of stenosis. The use of the linear stapler for an application as previously described overcomes a deep-rooted disadvantage which previously prevented its application. In particular, the advantageous prevision of using the exemplary linear stapler for carrying out of both anastomoses in a method as described permits maintaining good blood perfusion of the affected tissues and permits having available an instrument of limited size adapted to operate in a restricted area of the abdomen.

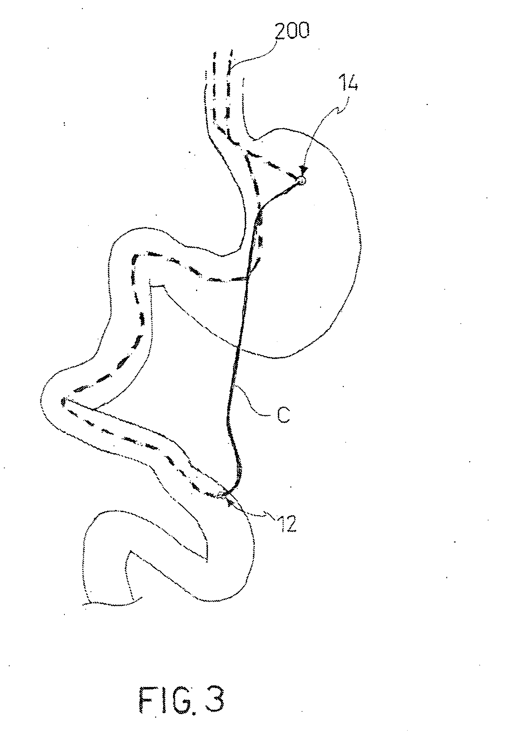

In accordance with a preferred embodiment, the steps of bringing close together tissues and/or creating the gastroenteroanastomosis and/or enteroenteroanastomosis may be realised intraluminally by using an anastomotic device as for example illustrated in figures 3-6. Such device is preferably made to slide along a guide means, preliminarily inserted through the portions to be brought together and/or joined and making up part of the instrumentation according to the present invention. Preferably, the guide means comprises at least one guide cable 200 arranged as an open loop which crosses through the portions to be joined and which can be associated with an anastomotic device.

The partial realisation of a pouch 100 in the stomach may be previously foreseen, to which the first intestine portion will be connected. Subsequently, a first guide cable open loop C is realised through the open portion of the gastric pouch and through the first portion 12 of the intestine and the second portion 14 of the stomach to be joined. In accordance with one possible embodiment, the gastric pouch is realised at the end of the procedure, after the realisation of the gastroenteroanastomosis and the enteroenteroanastomosis. In such case, the first guide cable open loop C is realised through the stomach and the intestine, crossing the tissue portions to be united as for example illustrated in figure 3.

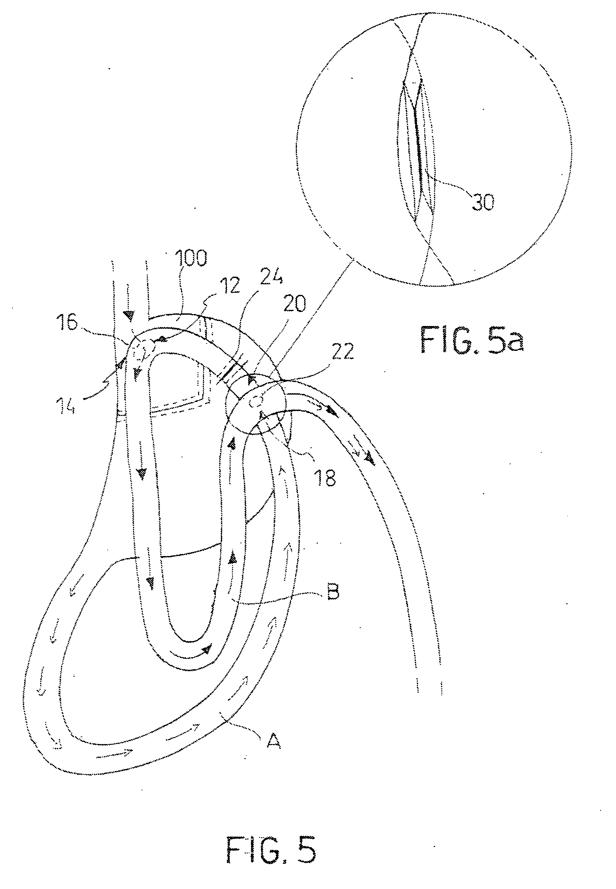

An anastomotic device is inserted and locked on the guide means, and transported, by means of the guide means itself, until it abuts against the first portion 12 to be joined and brings it close to the second portion 14 to be joined. This first sequence of steps concludes with the realisation of a gastroenteroanastomosis. The anastomotic device can be realised by means of an anvil adapted to be locked on the guide cable and adapted to cooperate with a circular stapler to carry out the gastroenteroanastomosis. Alternatively, the anastomotic device can be a device, which does not form part of the invention, adapted to position an anastomotic ring, preferably elastic, to keep the two tissue portions joined (as for example illustrated in figure 5a with reference to the enteroenteroanastomosis).

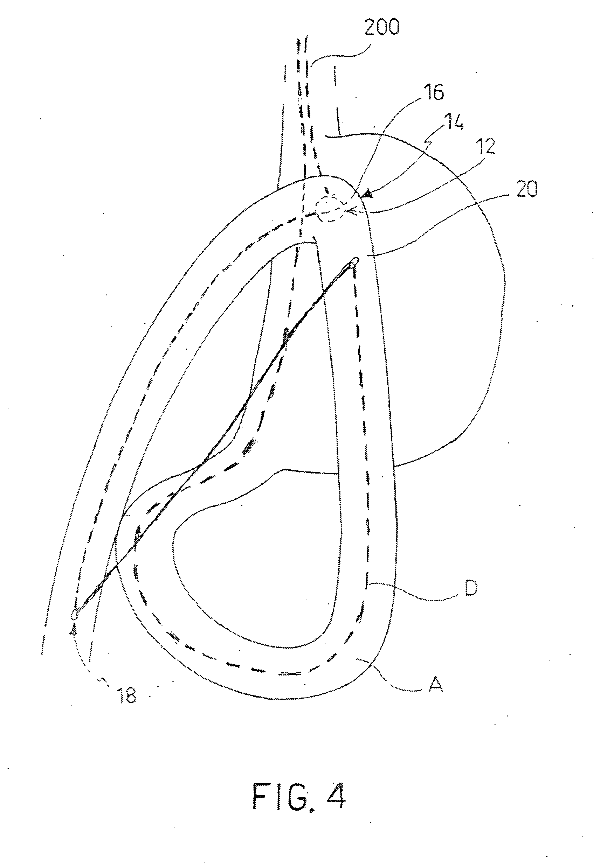

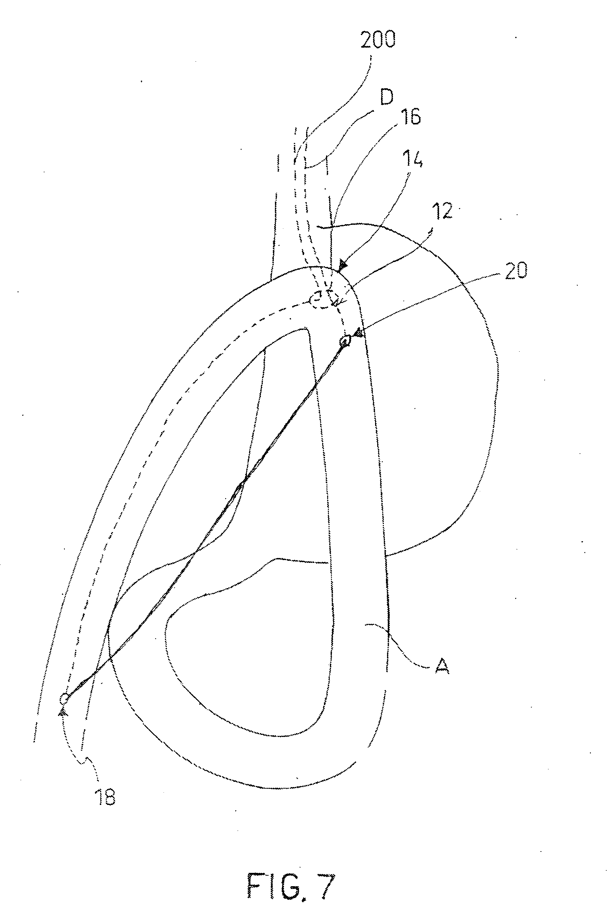

Subsequently, a second guide cable open ring D may be realised through the two portions of the intestine to be joined (additional first portion 18, distal with respect to the gastroenteroanastomosis, and additional second portion 20, proximal with respect to the gastroenteroanastomosis). If the gastric pouch is partially realised at the beginning of the procedure, the second guide cable open loop also crosses the open portion of the gastric pouch. Figure 4 illustrates the second guide cable open loop D without preliminary, partial formation of the gastric pouch. The second guide cable open loop crosses the gastroenteroanastomosis 16, the additional first portion 18, distal with respect to the gastroenteroanastomosis 16 with reference to the natural flow of the intestine duct, the additional second portion 20, proximal with respect to the gastroenteroanastomosis, and has the two ends preferably at the same orifice. Figure 7 illustrates a possible variation wherein the guide cable open loop D may be realised by crossing the gastroenteroanastomosis 16, the additional first portion 18, distal with respect to the gastroenteroanastomosis 16 with reference to the natural flow of the intestine duct, the additional second portion 20, proximal with respect to the gastroenteroanastomosis, and has the two ends in correspondence preferably with the same orifice.

Also in this case, an anastomotic device is inserted and locked on the guide means, and transported, by means of the guide means itself, until it abuts against the additional first portion 18 to be joined and brings it close to the additional second portion 20 to be joined. This second sequence of steps concludes with the realisation of an enteroenteroanastomosis and the completion of the gastric pouch. As with the gastroenteroanastomosis, the anastomotic device can be realised by means of an anvil adapted to be locked on the guide cable and adapted to cooperate with a circular stapler to carry out the enteroenteroanastomosis. Alternatively, the anastomotic device, which does not form part of the invention, can be a device adapted to position an anastomotic ring 30, preferably elastic, to keep the two tissue portions joined.

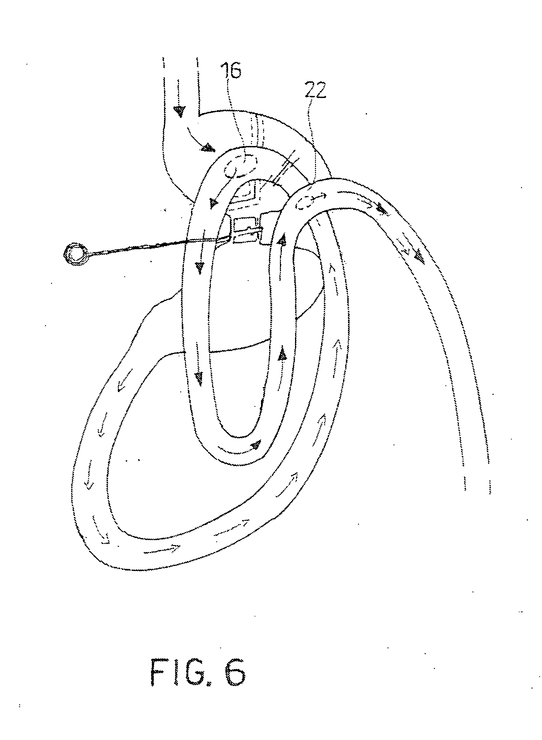

After having realised the gastroenteroanastomosis and the enteroenteroanastomosis, it is possible to simultaneously test both as previously described. Preferably, the step of the seal test may be carried out after the realisation (or completion) of the gastric pouch. Finally, the intestine may be sectioned between the gastroenteroanastomosis and the enteroenteroanastomosis with the section line 24. Figure 5 illustrates this latter situation wherein also the realisation of the gastric pouch 100 is highlighted at the end of the procedure. Figure 5a illustrates a detail of figure 5 wherein a possible formation is highlighted of the enteroenteroanastomosis by means of an exemplary elastic loop released by an anastomotic positioner device. The same solution may possibly be adopted for the gastroenteroanastomosis 16. Figure 6 illustrates another exemple embodiment wherein a gastric bandage is realised.

The passage of the guide means through the walls of the tissues to be united can be realised by perforating the wall (for example with radiofrequency needles) at the zone intended to form the anastomosis, so that after the formation of the anastomosis the continuity of the intestinal duct is restored.

The guide means and the anastomotic device as previously described may be used in any technique, for example hybrid intraluminal and laparoscopic or other type.

The instrumentation according to the present invention foresees means for bringing close together the tissue portions comprising an anastomotic device adapted to bring close together and/or join the tissues intraluminally.

An anastomotic device adapted for such purpose is a circular stapler sliding on the guide means and cooperating with an anvil, lockable on the guide means.

The instrumentation according to the present invention comprises means for partially realising a gastric pouch before inserting the guide means, wherein the second tissue portion 14 of the stomach is arranged at the gastric pouch, or means for realising the complete gastric pouch at the end of the procedure. In the first case, means are advantageously foreseen for completing the gastric pouch after the formation of the gastroenteroanastomosis and enteroenteroanastomosis.

The exemplary method and the instrumentation described above with reference to a procedure with guide means, preferably intraluminally, may be applied both to the step of carrying out the gastroenteroanastomosis and the step of carrying out the enteroenteroanastomosis, or to one of these.

As in the previous case, the foreseen exemplary method and instrumentation allow reducing the risks of mortality in gastrointestinal bypasses and considerably limiting the operation times. The maintenance of the continuity in the intestine until the completion of the two anastomosis permits the simultaneous verification of both. Moreover, due to the close arrangement of the two anastomoses, the operation area is limited to the upper zone of the abdomen.

A man skilled in the art, in order to satisfy specific and contingent needs, can make numerous modifications and adaptations to the preferred embodiments of the devices described above, as well as substitute elements with other functionally equivalent ones, without however departing from the scope of the following claims.

## Claims

1. Instrumentation for carrying out a gastrointestinal bypass comprising:
- means for bringing a first tissue portion (12) of the intestine close to a second tissue portion (14) of the stomach, realising a first loop (A) of the intestine between the stomach and the first tissue portion,
- means for creating a gastroenteroanastomosis (16) between the two close tissue portions, maintaining or restoring the continuity of the intestinal duct after the realisation of the gastroenteroanastomosis,
- means for bringing close together an additional first tissue portion (18) of the intestine arranged distally or downstream with respect to the gastroenteroanastomosis (16) with reference to the natural flow along the intestinal duct, and an additional second tissue portion (20) of the intestine arranged proximally, or upstream, with respect to the gastroenteroanastomosis (16) with reference to the natural flow along the intestinal duct, realising a second loop (B) of the intestine which is distal with respect to the gastroenteroanastomosis (16), wherein said additional second tissue portion (20) of the intestine, proximal with respect to the gastroenteroanastomosis (16), is brought close to the gastroenteroanastomosis (16),
- means for creating an enteroenteroanastomosis (22) between the two close tissue portions of the intestine, maintaining or restoring the continuity of the intestinal duct after the realisation of the enteroenteroanastomosis,
- means for sectioning the intestine between the gastroenteroanastomosis (16) and the enteroenteroanastomosis (22),
wherein said means for creating said gastroenteroanastomosis (16) and said means for enteroenteroanastomosis (22) comprises a circular stapler and an anvil,
**characterized in that:**
said means (200) for realizing said first loop (A) and said second loop (B) of the intestine comprises a guide cable (200) configured and having a length such as to define:
- a first guide cable open loop (C) adapted to pass through the esophagus and the stomach and the intestine until said first tissue portion (12) thereof and across the wall of said first tissue portion (12) to said second tissue portion (14) of the stomach and across the wall of said second tissue portion (14) of the stomach into the stomach and back through the esophagus and
- a second guide cable open loop (D) adapted to pass through the esophagus and the stomach and the intestine until said additional second tissue portion (20) of the intestine upstream said first tissue portion (12) and across the wall of said second tissue portion (20) to said additional first tissue portion (18) of the intestine downstream said first tissue portion (12) thereof and across the wall of said additional first tissue portion (18) into the intestine and further passing through the intestine until and across said gastroenteroanastomosis (16) into the stomach and back through the esophagus, and **in that** said means for creating said gastroenteroanastomosis (16) and said enteroenteroanastomosis (22) are further configured **in that**:
- the said circular stapler is configured to slide on said guide cable (200) and,
- the said anvil is adapted to be locked on said guide cable (200) and adapted to cooperate with said circular stapler for bringing said tissues close together intraluminally and carrying out said gastroenteroanastomosis (16) and said enteroenteroanastomosis (22) intraluminally,
wherein said anvil is transportable by means of said guide cable (200) itself along said first guide cable open loop (C) to said first tissue portion (12) and along said second guide cable open loop (D) to said additional first tissue portion (18), respectively.

2. Instrumentation according to claim 1, comprising means for carrying out a seal test both of the gastroenteroanastomosis (16) and the enteroenteroanastomosis (22), before the intestine is sectioned between the gastroenteroanastomosis (16) and the enteroenteroanastomosis (22).

3. Instrumentation according to claim 2, wherein the means for carrying out the seal test are adapted to simultaneously test both the gastroenteroanastomosis (16) and the enteroenteroanastomosis (22).

4. Instrumentation according to claim 1, comprising means for partially making a gastric pouch before inserting said guide cable (200), said second tissue portion (14) of the stomach being arranged at the gastric pouch.

5. Instrumentation according to claim 4, comprising means for completing the gastric pouch after the formation of the gastroenteroanastomosis and enteroenteroanastomosis,

6. Instrumentation according to claim 4, comprising means for making a gastric pouch after the formation of the gastroenteroanastomosis (16) and the enteroenteroanastomosis (20), said second tissue portion (14) of the stomach being arranged at the gastric pouch.

## Patentansprüche

1. Instrumentierung zur Durchführung eines gastrointestinalen Bypasses, umfassend:
- Mittel, um einen ersten Gewebeabschnitt (12) des Darms nahe zu einem zweiten Gewebeabschnitt (14) des Magens zu bringen, um eine erste Schleife (A) des Darms zwischen dem Magen und dem ersten Gewebeabschnitt zu realisieren,
- Mittel zur Erzeugung einer Gastroenteroanastomose (16) zwischen den beiden nahen Gewebeabschnitten, um die Kontinuität des intestinalen Duktus nach der Realisierung der Gastroenteroanastomose beizubehalten oder wiederherzustellen,
- Mittel, um einen zusätzlichen ersten Gewebeabschnitt (18) des Darms, der distal oder stromabwärts bezüglich der Gastroenteroanastomose (16) mit Bezug zum natürlichen Fluss entlang des intestinalen Duktus angeordnet ist, und einen zusätzlichen zweiten Gewebeabschnitt (20) des Darms, der proximal oder stromaufwärts bezüglich der Gastroenteroanastomose (16) mit Bezug zum natürlichen Fluss entlang des intestinalen Duktus angeordnet ist, nahe zueinander zu bringen, um eine zweite Schleife (B) des Darms, die distal bezüglich der Gastroenteroanastomose (16) ist, zu realisieren, wobei der zweite zusätzliche Gewebeabschnitt (20) des Darms, der proximal bezüglich der Gastroenteroanastomose (16) ist, nahe an die Gastroenteroanastomose (16) gebracht wird,
- Mittel zur Erzeugung einer Enteroenteroanastomose (22) zwischen den beiden nahen Gewebeabschnitten des Darms, um die Kontinuität des intestinalen Duktus nach der Realisierung der Enteroenteroanastomose beizubehalten oder wiederherzustellen,
- Mittel zur Aufteilung des Darms zwischen der Gastroenteroanastomose (16) und der Enteroenteroanastomose (22), wobei das Mittel zur Erzeugung der Gastroenteroanastomose (16) und das Mittel für die Enteroenteroanastomose (22) einen zirkularen Hefter und einen Amboss aufweist, **dadurch gekennzeichnet dass** das Mittel (200) zur Realisierung der ersten Schleife (A) und der zweiten Schleife (B) des Darms ein Führungskabel (200) aufweist, das konfiguriert ist und eine Länge aufweist, um zu definieren:
- eine erste offene Schleife (C) des Führungskabels, die eingerichtet ist, durch den Ösophagus und den Magen und den Darm bis zum ersten Gewebeabschnitt (12) desselben und durch die Wand des ersten Gewebeabschnitts (12) zum zweiten Gewebeabschnitt (14) des Magens und durch die Wand des zweiten Gewebeabschnitts (14) des Magens in den Magen und zurück durch den Ösophagus hindurchzugehen und
- eine zweite offene Schleife (D) des Führungskabels, die eingerichtet ist, durch den Ösophagus und den Magen und den Darm bis zum zusätzlichen zweiten Gewebeabschnitt (20) des Darms stromaufwärts des ersten Gewebeabschnitts (12) und durch die Wand des zweiten Gewebeabschnitts (20) zum zusätzlichen ersten Gewebeabschnitt (18) des Darms stromabwärts des ersten Gewebeabschnitts (12) desselben und durch die Wand des zusätzlichen ersten Gewebeabschnitts (18) in den Darm hindurchzugehen und darüber hinaus durch den Darm bis und durch die Gastroenteroanastomose (16) in den Magen und zurück durch den Ösophagus hindurchzugehen,
und **dadurch**, dass das Mittel zur Erzeugung der Gastroenteroanastomose (16) und der Enteroenteroanastomose (22) weiter **dadurch** konfiguriert sind, dass:
- der zirkulare Hefter konfiguriert ist, auf dem Führungskabel (200) zu gleiten und
- der Amboss eingerichtet ist, auf dem Führungskabel (200) verriegelt zu werden und eingerichtet ist, mit dem zirkularen Hefter zusammenzuwirken, um die Gewebe intraluminal nahe zueinander zu bringen und die Gastroenteroanastomose (16) und die Enteroenteroanastomose (22) intraluminal durchzuführen,
wobei der Amboss mittels des Führungskabels (200) selbst entlang der ersten offenen Schleife (C) des Führungskabels zum ersten Gewebeabschnitt (12) beziehungsweise entlang der zweiten offenen Schleife (D) des Führungskabels zum zusätzlichen ersten Gewebeabschnitt (18) transportierbar ist.

2. Instrumentierung nach Anspruch 1, welches Mittel zur Duchführung eines Dichtigkeitstests sowohl der Gastroenteroanastomose (16) als auch der Enteroenteroanastomose (22) aufweist, bevor der Darm zwischen der Gastroenteroanastomose (16) und der Enteroenteroanastomose (22) aufgeteilt ist.

3. Instrumentierung nach Anspruch 2, wobei das Mittel zur Durchführung des Dichtigkeitstests eingerichtet ist, gleichzeitig sowohl die Gastroenteroanastomose (16) als auch die Enteroenteroanastomose (22) zu testen.

4. Instrumentierung nach Anspruch 1, welches Mittel aufweist, um partiell eine Magentasche vor dem Einführen des Führungskabels (200) herzustellen, wobei der zweite Gewebeabschnitt (14) des Magens an der Magentasche angeordnet ist.

5. Instrumentierung nach Anspruch 4, das Mittel zum Vervollständigen der Magentasche nach der Bildung der Gastroenteroanastomose und der Enteroenteroanastomose aufweist.

6. Instrumentierung nach Anspruch 4, das Mittel zum Herstellen einer Magentasche nach der Bildung der Gastroenteroanastomose (16) und der Enteroenteroanastomose (22) aufweist, wobei der zweite Gewebeabschnitt (14) des Magens an der Magentasche angeordnet ist.

## Revendications

1. Instrumentation pour réaliser une dérivation gastro-intestinale comprenant :
✔ des moyens pour amener une première partie de tissu (12) de l'intestin à proximité d'une deuxième partie de tissu (14) de l'estomac, réalisant une première boucle (A) de l'intestin entre l'estomac et la première partie de tissu,
✔ des moyens pour créer une gastroentéroanastomose (16) entre les deux parties de tissu proches, conservant ou rétablissant la continuité du conduit intestinal après la réalisation de la gastroentéroanastomose,
✔ des moyens pour rapprocher une première partie de tissu supplémentaire (18) de l'intestin agencée de manière distale ou en aval par rapport à la gastroentéroanastomose (16) en référence au flux naturel le long du conduit intestinal, et une deuxième partie de tissu supplémentaire (20) de l'intestin agencée de manière proximale ou en amont par rapport à la gastroentéroanastomose (16) en référence au flux naturel le long du conduit intestinal, réalisant une deuxième boucle (B) de l'intestin qui est distale par rapport à la gastroentéroanastomose (16), dans laquelle ladite deuxième partie de tissu supplémentaire (20) de l'intestin, proximale par rapport à la gastroentéroanastomose (16), est amenée à proximité de la gastroentéroanastomose (16),
✔ des moyens pour créer une entéro-entéroanastomose (22) entre les deux parties de tissu proches de l'intestin, conservant ou rétablissant la continuité du conduit intestinal après la réalisation de l'entéro-entéroanastomose,
✔ des moyens pour sectionner l'intestin entre la gastroentéroanastomose (16) et l'entéro-entéro-anastomose (22),
dans laquelle lesdits moyens pour créer ladite gastroentéroanastomose (16) et lesdits moyens pour l'entéro-entéro-anastomose (22) comprennent une agrafeuse circulaire et une enclume,
**caractérisée en ce que** :
lesdits moyens (200) pour réaliser ladite première boucle (A) et ladite deuxième boucle (B) de l'intestin comprennent un câble de guidage (200) configuré et ayant une longueur afin de définir :
✔ une première boucle ouverte (C) de câble de guidage adaptée pour passer à travers l'oesophage et l'estomac et l'intestin jusqu'à sa première partie de tissu (12) et sur la paroi de ladite première partie de tissu (12) jusqu'à ladite deuxième partie de tissu (14) de l'estomac et sur la paroi de ladite deuxième partie de tissu (14) de l'estomac dans l'estomac et ressortir par l'oesophage, et
✔ une deuxième boucle ouverte (D) de câble de guidage adaptée pour passer à travers l'oesophage et l'estomac et l'intestin jusqu'à ladite deuxième partie de tissu supplémentaire (20) de l'intestin en amont de ladite première partie de tissu (12) et sur la paroi de ladite deuxième partie de tissu (20) jusqu'à ladite première partie de tissu supplémentaire (18) de l'intestin en aval de sa première partie de tissu (12) et sur la paroi de ladite première partie de tissu (18) dans l'intestin et passant en outre par l'intestin jusque et sur ladite gastroentéroanastomose (16) dans l'estomac et ressortir par l'oesophage,
et **en ce que** lesdits moyens pour créer ladite gastroentéroanastomose (16) et ladite entéro-entéro-anastomose (22) sont en outre configurés en ce que :
✔ ladite agrafeuse circulaire est configurée pour coulisser sur ledit câble de guidage (200), et
✔ ladite enclume est adaptée pour être bloquée sur ledit câble de guidage (200) et adaptée pour coopérer avec ladite agrafeuse circulaire pour rapprocher lesdits tissu de manière intraluminale et réaliser ladite gastroentéroanastomose (16) et ladite entéro-entéroanastomose (22) de manière intraluminale,
dans laquelle ladite enclume est transportable au moyen dudit câble de guidage (200) lui-même le long de ladite première boucle ouverte (C) de câble de guidage jusqu'à ladite première partie de tissu (12) et le long de ladite deuxième boucle ouverte (D) de câble de guidage jusqu'à ladite première partie de tissu supplémentaire (18), respectivement.

2. Instrumentation selon la revendication 1, comprenant des moyens pour réaliser un test d'étanchéité à la fois pour la gastroentéroanastomose (16) et l'entéro-entéro-anastomose (22), avant que l'intestin ne soit sectionné entre la gastroentéroanastomose (16) et l'entéro-entéro-anastomose (22).

3. Instrumentation selon la revendication 2, dans laquelle les moyens pour réaliser le test d'étanchéité sont adaptés pour tester simultanément à la fois la gastroentéroanastomose (16) et l'entéro-entéro-anastomose (22).

4. Instrumentation selon la revendication 1, comprenant des moyens pour réaliser partiellement une poche gastrique avant d'insérer ledit câble de guidage (200), ladite deuxième partie de tissu (14) de l'estomac étant agencée au niveau de la poche gastrique.

5. Instrumentation selon la revendication 4, comprenant des moyens pour achever la poche gastrique après la formation de la gastroentéroanastomose et de l'entéro-entéro-anastomose.

6. Instrumentation selon la revendication 4, comprenant des moyens pour réaliser une poche gastrique après la formation de la gastroentéroanastomose (16) et de l'entéro-entéro-anastomose (20), ladite deuxième partie de tissu (14) de l'estomac étant agencée au niveau de la poche gastrique.
